# DEMANDE DE BREVET EUROPEEN

(11) **EP 1 932 564 A1**
(43) Date de publication de la demande: **18.06.2008**
(21) Numéro de dépôt: 07121487.8
(22) Date de dépôt: 26.11.2007
(51) Int. Cl.: A61Q 19/00, A61Q 19/08, A61K 8/368, A61K 8/37

(54) **Utilisation cosmétique de l'acide anisique pour favoriser la desquamation**

(30) Priorité: 12.12.2006 FR 0655445
(71) Demandeur: L'Oréal, 75008 Paris (FR)
(72) Inventeur: Cotton, Thierry, 75013, Paris (FR); Amar, David, 75015, Paris (FR)
(74) Mandataire: Leonard, Armelle

(57) **Abrégé**

La présente invention porte sur l'utilisation cosmétique d'une composition contenant, dans un milieu physiologiquement acceptable, de l'acide anisique, l'un de ses sels ou l'un de ses esters d'alkyle en C1-C4, pour favoriser la desquamation de la peau et/ou stimuler le renouvellement épidermique.

L'invention porte également sur l'utilisation cosmétique de l'acide anisique, l'un de ses sels ou l'un de ses esters d'alkyle en C1-C4 dans une composition contenant un milieu physiologiquement acceptable, comme agent destiné à favoriser la desquamation de la peau et/ou stimuler le renouvellement épidermique.

L'acide anisique, l'un de ses sels ou l'un de ses esters d'alkyle en C1-C4 ou la composition le contenant est notamment destiné à améliorer l'éclat du teint et/ou le grain de la peau et/ou lisser le microrelief cutané, et/ou atténuer les tâches de vieillesse.

## Description

La présente invention se rapporte à l'utilisation cosmétique d'une composition contenant, dans un milieu physiologiquement acceptable, de l'acide anisique ou l'un de ses dérivés, pour favoriser la desquamation de la peau et/ou stimuler le renouvellement épidermique.

L'invention porte également sur l'utilisation cosmétique de l'acide anisique ou l'un de ses dérivés dans une composition contenant un milieu physiologiquement acceptable, comme agent destiné à favoriser la desquamation de la peau et/ou stimuler le renouvellement épidermique.

L'acide anisique ou l'un de ses dérivés ou la composition le contenant est notamment destiné à améliorer l'éclat du teint et/ou le grain de la peau et/ou lisser le microrelief cutané, et/ou atténuer les tâches de vieillesse.

Par 'dérivé de l'acide anisique' au sens de la présente invention, on entend un sel d'acide anisique ou un ester d'alkyle en C₁₋₄ de l'acide anisique.
En particulier, il s'agira d'un sel de métal alcalin ou alcalino-terreux, d'un sel d'ammonium ou d'un sel avec une amine organique, ou encore d'un ester d'alkyle en C₁₋₄.
Ces dérivés n'incluent pas les dérivés hydroxy- de l'acide methoxybenzoïque, tels que ceux décrits dans les demandes FR2739556, US2002028254 et US5766613.

Il peut notamment s'agir de compositions de soin et/ou de nettoyage de la peau.

Par « peau », on entend au sens large la peau et les semi-muqueuses (lèvres).

Cette composition est notamment destinée au soin et/ou au nettoyage de la peau, en particulier au soin et/ou au nettoyage des peaux âgées, ou des peaux à l'aspect de teint terne et/ou rugueux.

La desquamation est un phénomène naturel lié au fait que l'épiderme, qui constitue la couche supérieure de la peau et du cuir chevelu, est en constante régénération.
L'épiderme est constitué de plusieurs assises de cellules, dont la plus profonde est l'assise basale constituée de cellules indifférenciées. Au cours du temps, ces cellules vont se différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement.

Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée *stratum corneum.*

Il est connu que le processus de desquamation peut être altéré par des facteurs exogènes (ex : rayonnement UV, pollution, agents allergènes, pathogènes), et/ou endogènes (ex : changements hormonaux, âge...) et conduire notamment à un ralentissement du renouvellement épidermique et par conséquent un vieillissement de la peau, et/ou un épaississement de la couche cornée (ex : formation de callosités).
Des altérations du processus de desquamation peuvent également conduire à des désordres desquamatifs de type esthétique (ex : pellicules, squames...) ou pathologique (ex : xéroses, ichtyoses, psoriasis, dermatite atopique).

Pour ce qui est plus particulièrement du vieillissement cutané, résultant de facteurs intrinsèques ou extrinsèques, il se traduit généralement par une modification de l'aspect de la peau, qui peut se manifester par exemple par : une peau sèche, l'apparition de squames, l'aspect d'un teint brouillé, terne et/ou jaune, l'aspect d'une peau rugueuse et/ou d'une peau 'craquelée', l'apparition de rides et ridules, l'apparition de tâches de vieillesse, et/ou une visibilité accrue des pores.

Il existe donc le besoin de trouver des moyens permettant de favoriser la desquamation et/ou stimuler le renouvellement épidermique de la peau.

La Demanderesse vient de mettre en évidence que l'acide anisique, connu jusqu'à lors comme agent microbien, notamment dans des compositions capillaires de traitement du cuir cheveleu (EP1325731) ou dans des compositions déodorantes (WO87/06827), avait également des propriétés exfoliantes. Elle a en effet montré que l'acide anisique, appliqué topiquement sur un modèle de peau en survie, diminuait la cohésion du *stratum corneum* et favorisait ainsi le processus de desquamation et de renouvellement épidermique de la peau.

La présente invention se rapporte donc à l'utilisation cosmétique d'une composition contenant, dans un milieu physiologiquement acceptable, de l'acide anisique ou l'un de ses dérivés, pour favoriser la desquamation de la peau et/ou stimuler le renouvellement épidermique.

L'invention porte également sur l'utilisation cosmétique de l'acide anisique ou l'un de ses dérivés dans une composition contenant un milieu physiologiquement acceptable, comme agent destiné à favoriser la desquamation de la peau et/ou stimuler le renouvellement épidermique.

Autrement dit, ledit acide anisique ou l'un de ses dérivés ou ladite composition le contenant est destiné selon l'invention à favoriser la desquamation de la peau et/ou stimuler le renouvellement épidermique.

L'acide anisique (ou l'un de ses dérivés) en tant que tel et/ou la composition le contenant sont notamment destinés selon l'invention à améliorer au moins une des conditions choisie parmi :
- améliorer l'éclat et/ou l'homogénéité du teint et/ou diminuer l'aspect de teint brouillé et/ou terne ;
- améliorer l'aspect de surface, en particulier diminuer l'aspect rugueux ou craquelé de la peau et/ou améliorer le grain et/ou la douceur de la peau ;
- lisser le microrelief de la peau, en particulier lisser les ridules et les rides et/ou atténuer les marques d'acné ou de varicelle ;
- atténuer les tâches de vieillesse (ex : lentigo séniles) et/ou les désordres esthétiques de pigmentation, tels que les tâches pigmentaires brunâtres et/ou les éphélides ;
- diminuer la sécheresse de la peau, en particulier la sécheresse de la peau âgée ;
- améliorer l'apparence et/ou diminuer la visibilité des pores et/ou améliorer la désobstruction des pores ;
- favoriser l'action de nettoyage et l'élimination de cellules mortes à la surface de la peau ; et/ou
- lutter contre les imperfections des peaux grasses, en particulier l'aspect brillant ou luisant de la peau.

L'acide anisique (ou l'un de ses dérivés) en tant que tel et/ou la composition le contenant peuvent également être utilisés pour la préparation de compositions destinées au traitement de désordres de la pigmentation, tels que les mélasma, l'hyperpigmentation post-inflammatoire, les hyperpigmentations accidentelles, éventuellement dues à la photosensibilisation ou à la cicatrisation post-lésionnelle.

L'invention porte donc sur l'utilisation cosmétique d'une composition contenant, dans un milieu physiologiquement acceptable, de l'acide anisique ou l'un de ses dérivés, ou l'utilisation de l'acide anisique ou l'un de ses dérivés en tant que tel pour améliorer l'éclat et/ou l'homogénéité du teint et/ou diminuer l'aspect de teint brouillé et/ou terne.

Elle vise encore l'utilisation cosmétique d'une composition contenant, dans un milieu physiologiquement acceptable, de l'acide anisique ou l'un de ses dérivés, ou l'utilisation de l'acide anisique ou l'un de ses dérivés en tant que tel pour améliorer l'aspect de surface, en particulier diminuer l'aspect rugueux ou craquelé de la peau et/ou améliorer le grain et/ou la douceur de la peau.

Ou encore l'utilisation cosmétique d'une composition contenant, dans un milieu physiologiquement acceptable, de l'acide anisique ou l'un de ses dérivés, ou l'utilisation de l'acide anisique ou l'un de ses dérivés en tant que tel pour lisser le microrelief de la peau, en particulier lisser les ridules et les rides et/ou atténuer les marques d'acné ou de varicelle.

Elle porte encore sur l'utilisation cosmétique d'une composition contenant, dans un milieu physiologiquement acceptable, de l'acide anisique ou l'un de ses dérivés, ou l'utilisation de l'acide anisique ou l'un de ses dérivés en tant que tel pour atténuer les tâches de vieillesse.

Elle vise également l'utilisation cosmétique d'une composition contenant, dans un milieu physiologiquement acceptable, de l'acide anisique ou l'un de ses dérivés, ou l'utilisation de l'acide anisique ou l'un de ses dérivés en tant que tel pour diminuer la sécheresse de la peau, en particulier la sécheresse de la peau âgée.

Rentre également dans le cadre de l'invention l'utilisation cosmétique d'une composition contenant, dans un milieu physiologiquement acceptable, de l'acide anisique ou l'un de ses dérivés, ou l'utilisation de l'acide anisique ou l'un de ses dérivés en tant que tel pour améliorer l'apparence et/ou diminuer la visibilité des pores et/ou améliorer la désobstruction des pores.

L'invention couvre également l'utilisation cosmétique d'une composition contenant, dans un milieu physiologiquement acceptable, de l'acide anisique ou l'un de ses dérivés, ou l'utilisation de l'acide anisique ou l'un de ses dérivés en tant que tel pour favoriser l'action de nettoyage et l'élimination de cellules mortes à la surface de la peau

Elle porte encore sur l'utilisation cosmétique d'une composition contenant, dans un milieu physiologiquement acceptable, de l'acide anisique ou l'un de ses dérivés, ou l'utilisation de l'acide anisique ou l'un de ses dérivés en tant que tel pour lutter contre les imperfections des peaux grasses, en particulier l'aspect brillant ou luisant de la peau.

### ACIDE ANISIQUE et dérivés

L'acide anisique utilisé dans la présente invention peut être utilisé tel quel ou se trouver sous la forme d'un sel de métal alcalin ou alcalino-terreux, d'un sel d'ammonium ou d'un sel avec une amine organique, ou encore sous la forme d'un ester d'alkyle en C₁₋₄. Le groupe alkyle de l'ester peut être linéaire ou ramifié et on peut mentionner à titre d'exemples, les groupes méthyle, éthyle, n-propyle, isopropyle, n-butyle et tertio-butyle.
A titre d'exemples de sel ou d'ester d'acide anisique, on peut notamment citer l'anisate de sodium, l'anisate de potassium, l'anisate de méthyle, l'anisate d'éthyle, l'anisate de propyle ou l'anisate de butyle.
De préférence, on utilise l'acide anisique sous sa forme ortho-, meta-, ou para-, autrement nommé acide ortho-, meta-, paramethoxybenzoïque, et encore plus préférentiellement l'acide para-anisique ou l'acide 4-methoxybenzoïque.
En particulier, on pourra utiliser l'acide para-anisique ou l'acide 4-methoxybenzoïque ou acide para methoxybenzoïque commercialisé par la société MERCK.

Par 'dérivé de l'acide anisique' au sens de la présente invention, on entend un sel d'acide anisique ou un ester d'alkyle en C₁₋₄ de l'acide anisique.

En particulier, il s'agira d'un sel de métal alcalin ou alcalino-terreux, d'un sel d'ammonium ou d'un sel avec une amine organique, ou encore d'un ester d'alkyle en C₁₋₄.
Ces dérivés n'incluent pas les dérivés hydroxy- de l'acide methoxybenzoïque, tels que ceux décrits dans les demandes FR2739556, US2002028254 et US5766613.

L'acide anisique ou l'un de ses dérivés est notamment utilisé en une quantité pour obtenir l'effet desquamant recherché. A titre d'exemple, la quantité d'acide anisique ou l'un de ses dérivés dans la composition pourra aller de 0.001 à 50% en poids par rapport au poids total de la composition, de préférence de 0.01 à 5% en poids par rapport au poids total de la composition, encore plus préférentiellement de 0.05 à 2% en poids par rapport au poids total de la composition. De préférence encore, on utilisera une composition contenant de 0,1 à 0,5% en poids d'acide anisique ou l'un de ses dérivés par rapport au poids total de la composition.

Selon un mode particulier de l'invention, concernant les compositions de peeling, l'acide anisique ou l'un de ses dérivés pourra être utilisé dans lesdites compositions en une quantité allant de 1 à 50% en poids par rapport au poids total de la composition, de préférence de 1 à 30% en poids et encore plus préférentiellement de 5 à 20% en poids par rapport au poids total de la composition.

Les compositions contenant de l'acide anisique selon l'invention pourront être appliquées sur toute zone de la peau ou de ses annexes, notamment du visage, du corps, du décolleté, des mains, ou sur les lèvres.

Selon un mode particulier de l'invention, la composition est destinée au soin et/ou au nettoyage des peaux âgées.

Selon un autre mode, la composition est destinée au soin et/ou au nettoyage des peaux à l'aspect terne et/ou rugueux.

Selon encore un autre mode, la composition est destinée au soin et/ou au nettoyage des peaux grasses.

### GALENIQUE

Les compositions selon l'invention comprennent un milieu physiologiquement acceptable. Par « milieu physiologique acceptable », on entend désigner un milieu compatible avec la peau et les semi-muqueuses (lèvres) d'êtres humains.

Ce milieu physiologiquement acceptable comprend de l'eau, éventuellement en mélange ou non avec un ou plusieurs solvants organiques tels que des alcools en C₁-C₈, notamment l'éthanol, l'isopropanol, le tert-butanol, le n-butanol, des polyols comme la glycérine, le propylène glycol, le butylène glycol, le sorbitol et des éthers de polyol.

Les compositions selon l'invention peuvent être une composition cosmétique ou dermatologique et peuvent donc comprendre un milieu cosmétiquement ou pharmaceutiquement acceptable. De préférence, il s'agira d'une composition cosmétique destinée notamment au soin et/ou au nettoyage de la peau.

La composition peut également comprendre une phase grasse, qui peut comprendre des huiles, des gommes, des cires usuellement utilisées dans le domaine d'application considéré. Comme huiles ou cires utilisables dans l'invention, on peut citer les huiles minérales (huile de vaseline), les huiles végétales (fraction liquide du beurre de karité, huile de tournesol, huile d'abricot, de son de riz...), les huiles animales (perhydrosqualène), les huiles de synthèse (huile de Purcellin, l'isononanoate d'isononyle , le pentaerythrityl tetraethylhexanoate), les huiles ou cires siliconées (cyclométhicone, dimethicone , Phenylmethicone), les huiles fluorées (perfluoropolyéthers), les gommes de silicone (cyclopentasiloxane et dimethiconol), et polymères de silicone (type KSG, dimethicone vinyl dimethicone crosspolymer), les cires d'abeille, de carnauba, ou paraffine, le beurre de karité, l'huile de jojoba hydrogénée. On peut ajouter à ces huiles des alcools gras (cétyliques, stéaryliques, ...), des acides gras (acide stéarique, ....) ou des facteurs de consistance tels que le mélange de stéarate d'éthylène glycol acétyle, tri stéarate de glycéryle (Unitwix^{®}) .

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

La composition peut contenir également des adjuvants habituels dans le domaine considéré, tels que les tensioactifs, les émulsionnants, les gélifiants, les actifs hydrophiles ou lipophiles, les conservateurs, les antioxydants, les solvants, les parfums, les charges, des tenseurs (naturels et synthétiques), les filtres UV, les absorbeurs d'odeur et les matières colorantes, d'autres actifs cosmétiques ou pharmaceutiques.

Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme tensioactifs susceptibles d'être utilisés, on peut citer par exemple le stéarate de glycérol, le polysorbate 60 et le mélange de PEG-6/PEG-32/Glycol Stéarate vendu sous la dénomination de TefoseR 63 par la société Gattefosse ; des dérivés PEG stéarate, des dérivés de sucres, des esters de sucrose, des tensioactifs phosphatés (ex : Amphisol^{®}).

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, des copolymères réticulés ou non réticulés d'acide 2-acrylamido 2-méthylpropane sulfonique (AMPS), les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Comme filtres UV ou agents photoprotecteurs actifs dans l'UVA et/ou l'UVB utilisables dans la composition de l'invention, on peut citer notamment les agents photoprotecteurs organiques ou inorganiques.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; et les polymères filtres et silicones filtres tels que ceux décrits notamment dans la demande WO-93/04665 ; les dimères dérivés d'α-alkylstyrène tels que ceux décrits dans la demande de brevet DE19855649.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans la composition selon l'invention dans des proportions allant de 0,1 à 20 % en poids par rapport au poids total de la composition, et de préférence allant de 0,2 à 15 % en poids par rapport au poids total de la composition.

La composition peut se présenter sous toutes les formes galéniques envisageables adaptées à une application sur la peau.

Notamment, la composition peut avoir la forme d'une solution aqueuse, alcoolique, hydroalcoolique ou huileuse; d'une dispersion du type lotion ou sérum; d'une lotion multi-phases (ex : bi-phasée), d'une émulsion eau-dans-huile, huile-dans-eau ou multiple; d'une suspension; de microcapsules ou microparticules; de dispersions vésiculaires de type ionique et/ou non ionique; sous forme de sérum; d'une mousse, d'une préparation solide par exemple de stick; d'une composition pour aérosol comprenant également un agent propulseur sous pression ou sous forme d'un patch pouvant lui-même se présenter sous différentes formes galéniques.

Dans la famille des patchs, on peut citer notamment :
- des patchs à libération contrôlée avec une matrice polymérique hydrophobe tels que décrits par exemple dans le document FR 2 738 744 ;
- des patchs de type réservoir contenant un réservoir de substance active, une membrane de diffusion et une couche adhésive
- des patchs à effet de champ magnétique favorisant la pénétration des actifs dans la peau tels que décrits par exemple dans FR 2 791 750 ;
- des patchs à adhérence optimisée, comportant une couche de polymère hydrophobe fixée sur une couche support et contenant des particules de composé actif, des particules d'huile et des particules d'un agent hydro-absorbant tels que décrits par exemple dans FR2 761 889 ;
- des gels patchs à forte teneur en eau, de type hydrogel, notamment à base d'au moins un hydrocolloïde et/ou de polysaccharides, de protéines, de polyacrylamide, d'acide polyacrylique, de copolymères d'acide acrylique et d'amidon, de polyvinylpyrrolidone ou de dérivés d'acide polyvinylique, et présentant une adhésion de contact, similaire à celle résultant d'un effet ventouse.
- des gels patchs hydrophobes, aussi appelés organogels ou oléogels à base de polyéthylène, de silicones ou de copolymère de styrène et d'isoprène ou de copolymères de styrène et butadiène. Ces systèmes ont l'avantage de pouvoir contenir de fortes proportions de composés hydrophobes
- des gels patchs à base de de polyuréthanes segmentés (SPUG) dont le caractère lipophile ou hydrophile peut être modifié par modification des chaînes polyoxyéthylénées (Y. Shikinami, Adhesive polymer gels for medical uses, Kagaku To Tokyo, 67 (4), 141-150, 1993) ;

- des feuilles ou pastilles décrites dans FR 2 512 651 ou FR 2 538 247 ;
- des films composites tels que décrits dans EP-A- 0 285 563 ou US 2002/0187181 ; avantageusement, on pourra utiliser un film composite comportant une couche réservoir constituée par une matrice formée d'un polymère de silicone à l'intérieur de laquelle sont disposées des inclusions constituées par la phase aqueuse active gélifiée grâce à au moins un agent gélifiant (FR 2 650 747 L'Oreal);
- des films fins solides à base de polymères naturels ou synthétiques qui appliqués sur l'ongle préalablement mouillé se dissolvent in situ; de tels films sont décrits dans les demande de brevet WO 02/053197, WO 02/05789, WO 2004/032859, WO 02/054997 ;
- des films contenant un agent filmogène et un polymère adhésif hydrophile, aussi appelés, patch-non-patch, tels que décrits dans le brevet WO 02/30402 ;
- des compositions à base de polymères ayant des propriétés adhésives capables de former un film que l'on retire après séchage (EP- 0 514 760, EP-0 826 364) ou une couche solide élastique et pliable retirée en appliquant un tissu ou un plastique adhésif (WO93/05893) ; ou des gels ou pâtes qui, après application, sèchent pour donner un film que l'on retire par lavage, nettoyage ou arrachage ; on parlera généralement de 'vernis-patch' ou 'peel-patch'.

Mais on peut également utiliser des systèmes ou dispositifs augmentant l'absorption dudit acide anisique ou l'un de ses dérivés contenu dans la composition par un mécanisme actif (M.R. Prausnitz et al., Current status and future potential of transdermal drug delivery, Nature Rev., vol. 3, 115-124, 2004), comme par exemple :
- les systèmes d'injection sous pression ou sans-aiguille (Burkoth T.L. et al., Transdermal and transmucosal powdered drug delivery., Crit Rev Ther Drug Carrier Syst. 1999;16(4):331-84 ;
- l'utilisation d'ultra-sons : Sonophorèse et phonophorèse (Joshi A. and Raje J., Sonicated transdermal drug transport, J. Control. Release, 83 (1), 13-22, 2002) ;
- l'utilisation de courants électriques : lontophorèse, electroporation, courant microfréquence (Riviere J.E. and Heit M.C., Electrically-assisted transdermal drug delivery, Pharm. Res. 14 (6), 687-697, 1997) (brevets US 6,148,232, WO 03/035167) ;
- l'utilisation d'ondes magnétiques : magnétophorèse (Murthy S.N., Magnetophoresis: an approach to enhance transdermal drug diffusion., Pharmazie. 1999 May;54(5):377-9 ) ;
- l'utilisation de micro-aiguilles ( Mc Allister D.V. et al., Microfabricated microneedles for gene and drug delivery, Annu. Rev. Biomed. Eng., 2, 289-313, 2000) (brevet US 6,451,240).

La composition selon l'invention peut se présenter sous la forme d'une composition de nettoyage ou de démaquillage, de protection solaire, de traitement ou de soin pour le visage, pour les mains, pour les pieds, pour les grands plis anatomiques ou pour le corps. A titre d'exemples, la composition peut être une crème de jour, une crème de nuit, une crème démaquillante, une composition SPF pour se protéger du soleil , un lait corporel de protection ou de soin, un lait après-solaire, une lotion, un gel ou mousse pour le soin de la peau, une lotion de nettoyage, une composition de bronzage artificiel, une crème ou mousse à raser.

Lorsque la composition selon l'invention est destinée au nettoyage et/ou au démaquillage de la peau, elle peut également se présenter sous la forme d'une solution ou d'un gel moussant ou d'une crème moussante avec ou sans savon ou d'une huile démaquillante. Selon ce mode particulier, la composition selon l'invention comprendra au moins un tensioactif, qui va apporter le caractère nettoyant, voire moussant, à la composition. Ce tensioactif peut être choisi parmi des tensioactifs non ioniques, des tensioactifs anioniques, des tensioactifs cationiques, des tensioactifs amphotères et zwitterioniques et des mélanges de ceux-ci.

Le tensioactif peut être présent dans une composition selon l'invention en une quantité allant de 0,1 à 50 % en poids, de préférence de 0,5 à 20 % en poids, en particulier de 1 à 15 % en poids, voire de 5 à 10 % en poids par rapport au poids total de la composition.
a) Les *tensioactifs non ioniques* peuvent être choisis par exemple parmi les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.
b) Les *tensioactifs anioniques* peuvent être choisis par exemple parmi les savons (sels alcalins d'acides gras), les carboxylates, les acylaminoacides, les amidoéther-carboxylates, les alkyl polyaminocarboxylates, les alkyl éthers sulfates tels que les Sodium Laureth sulfates, les alkyl sulfonates, les iséthionates, les alkyl méthyltaurates, les alkyl sulfosuccinates, les alkylsulfoacétates, les alkylphosphates (mono ou dialkylphosphates), leurs sels, et leurs mélanges.
c) Les *tensioactifs moussants amphotères et zwitterioniques* peuvent être choisis par exemple parmi les dérivés de bétaïnes dont les amidopropylbétaïnes, les amphoacétates et les amphodiacétates, les hydroxylsultaines et leurs mélanges.

Selon un mode de réalisation de l'invention, un tensioactif convenant à l'invention peut être choisi parmi les alkylpolyglucosides, les dérivés de bétaïne, les acides alkyl glycol carboxyliques et leurs sels, les alkyl éthers sulfates, les alkylphosphates, les amphodiacétates, les amphoacétates, les alkylglycinates, les acyls glutamates, les acyls sarcosinates et des mélanges de ceux-ci.

Selon un mode particulier, un tensioactif convenant à l'invention peut être choisi parmi le décyl glucoside, le cocoyl glucoside, le lauryl éthersulfate de sodium, le cocoyl bétaïne, la lauroyl bétaïne, la cocamidopropyl bétaïne, la lauramido propylbétaïne, le lauryl glycol carboxylate, le cocoampho(di)acétate, le lauroampho(di)acétate, le lauryl phosphate de potassium, un mélange de ceux-ci.

Comme tensioactifs préférés, on pourra citer notamment des acides gras ; des polysorbates (ex: polysorbate 85) ; les PEG-stéarates et isostearates tels que le PEG-20 glyceryl stéarate ; le N Cocoylglycinate de Na; et l'alcool behenique Oxyéthyléné .

Lorsque la composition selon l'invention est destinée à un usage de type peeling , elle peut également se présenter sous toutes les formes galéniques évoquées ci-dessus pour autant qu'elle s'élimine facilement par rinçage, et notamment sous forme de gel aqueux, de solution aqueuse ou hydroalcoolique. Elle peut être appliquée par tout moyen permettant une répartition uniforme et notamment à l'aide d'un coton, d'une tige, d'un pinceau, d'une gaze, d'une spatule ou d'un tampon, ou encore par pulvérisation, et peut être éliminée par rinçage à l'eau ou à l'aide d'un détergent doux. Selon un mode préféré de réalisation de l'invention, la composition destinée au peeling chimique renferme une phase aqueuse continue.

Selon un mode avantageux de l'invention, les propriétés desquamantes de l'acide anisique pourront être associées à d'autres agents cosmétiques de soin de la peau.

En particulier, la composition selon l'invention pourra comprendre en outre au moins un autre actif cosmétique choisi parmi d'autres agents desquamants, des agents hydratants, des agents antioxydants, des agents dépigmentants, des agents stimulant le métabolisme énergétique des cellules, des agents stimulant la prolifération ou la différenciation épidermique et/ou la synthèse de macromolécules épidermiques ou dermiques, des agents diminuant ou inhibant l'activité de protéases néfastes, des agents favorisant la fonction barrière cutanée, des agents tenseurs, des agents apaisants, des agents antiséborrhéiques, et leurs mélanges.

Selon un premier mode, la composition selon l'invention comprendra, outre l'acide anisique ou l'un de ses sels, au moins un autre agent desquamant.
Selon une autre alternative, la composition selon l'invention comprendra, outre l'acide anisique ou l'un de ses sels, au moins un agent antioxydant.
Selon un autre mode, la composition selon l'invention comprendra, outre l'acide anisique ou l'un de ses sels, au moins un agent dépigmentant.
Selon un autre mode, la composition selon l'invention comprendra, outre l'acide anisique ou l'un de ses sels, au moins un agent stimulant le métabolisme énergétique des cellules. Selon un autre mode, la composition selon l'invention comprendra, outre l'acide anisique ou l'un de ses sels, au moins un agent stimulant la prolifération ou la différenciation épidermique et/ou la synthèse de macromolécules épidermiques ou dermiques.
Selon un autre mode, la composition selon l'invention comprendra, outre l'acide anisique ou l'un de ses sels, au moins un agent diminuant ou inhibant l'activité de protéases néfastes.
Selon un autre mode, la composition selon l'invention comprendra, outre l'acide anisique ou l'un de ses sels, au moins un agent favorisant la fonction barrière cutanée.
Selon un autre mode, la composition selon l'invention comprendra, outre l'acide anisique ou l'un de ses sels, au moins un agent tenseur.
Selon une alternative, la composition selon l'invention comprendra, outre l'acide anisique ou l'un de ses sels, au moins un agent antiséborrhéique.

La composition pourra comprendre en outre un agent hydratant.
Selon un autre mode, elle pourra comprendre en outre un agent apaisant.

Des exemples de tels agents sont cités ci-dessous.
On peut citer notamment :
1) d'autres agents desquamants, tels que :
   - les α-hydroxyacides, tels que les acides glycolique, citrique, lactique, tartrique, malique ou mandélique ; l'acide gentisique et ses dérivés ; l'acide cinnamique ; l'acide jasmonique et dérivés comme dans les demandes de brevet EP 1 333 022 et EP 1 333021;
   - les β-hydroxyacides, en particulier l'acide salicylique et ses dérivés dont l'acide n-octanoyl 5-salicylique connu sous le nom INCl : Capryloyl salicylic Acid ;
   - l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M^{®}); le (3-hydroxy-2pentylcyclopentyl)acetic acid ; l'acide azélaïque ; l'acide nicotinique et ses dérivés ; l'acide ascorbique et ses dérivés, tels que le glucoside d'ascorbyle et l'ascorbyl phosphate de magnésium ;
   - l'urée et ses dérivés, tels que ses dérivés hydroxyalkyl et en particulier le N-(2-hydroxyéthyl)-urée disponible dans le commerce, sous forme de mélange à 50% en poids dans l'eau, auprès de la société NATIONAL STARCH sous la dénomination commerciale Hydrovance^{®};
   - l'acide 8-hexadécène-1,16-dicarboxylique ou acide 9-octadécène dioïque autrement nommé acide dioïque, et ses dérivés ; l'acide dioïque est notamment disponible dans le commerce auprès de la société UNIQEMA sous la dénomination commerciale Arlatone Dioic DCA^{®}.
   L'utilisation de l'acide anisique en association avec d'autres agents desquamants permet avantageusement de diminuer la quantité d'agents desquamants efficace pour obtenir l'effet recherché.
2) des agents hydratants, par lesquels on entend :
   - soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
   - soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ; les dérivés C-glycosides tels que ceux décrits dans EP 1 345 919, en particulier le C-β-D-xylopyranoside-2-hydroxy-propane commercialisé par la société Chimex sous la dénomination Mexoryl SBB^{®} (nom INCI : hydroxypropyl tetrahydropyrantriol) ; un extrait de *Laminaria ochroleuca* tel que la Laminaïne^{®} de Secma stimulant la synthèse des glycosaminoglycanes ;
   - soit un composé activant les glandes sébacées tel que les dérivés stéroïdiens (dont la DHEA, ses dérivés 7-oxydés et/ou 17-alkylés et les sapogénines), le dihydrojasmonate de méthyle, et la vitamine D et ses dérivés.
3) des agents antioxydants ou anti-radicalaires, tels que les polyphénols ; la vitamine E et ses dérivés, en particulier l'acétate de vitamine E ; la vitamine A et ses esters, en particulier le palmitate de vitamine A ; les agents chélatants tels que l'EDTA ; des extraits de germe de blé à activité superoxyde dismutase, le phytantriol, les lignanes, l' Idebenone, la vitamine C, le coenzyme Q10, l'acide ellagique, la biotine.
4) des agents dépigmentants, tels que la vitamine C et ses dérivés et notamment la vit CG, CP et 3-O ethyl vitamine C, l'alpha et la béta arbutine, le lucinol et ses dérivés, l'acide kojique, le résorcinol et ses dérivés, l'acide tranexamique et ses dérivés, l'acide gentisique, l'homogentisate, le méthyl gentisate ou l'homogentisate, l'acide ellagique, la vitamine B3.
5) des agents stimulant le métabolisme énergétique des cellules tels que la biotine, un extrait de *Saccharomyces cerevisiae* tel que le Phosphovital^{®} de Sederma, le mélange de sels de sodium, de manganèse, de zinc et de magnésium d'acide pyrrolidone carboxylique comme le Physiogenyl^{®} de Solabia, un mélange de gluconate de zinc, de cuivre et de magnésium tel que le Sepitonic M3^{®} de Seppic et leurs mélanges.
6) des agents stimulant la prolifération ou la différenciation épidermique et/ou la synthèse de macromolécules dermiques ou épidermiques, tels que :
   - les agents stimulant la prolifération des kératinocytes, dont notamment les rétinoïdes tels que le rétinol et ses esters, dont le palmitate de rétinyle ; l'adénosine ; le phloroglucinol ; les extraits de tourteaux de noix commercialisés par la société GATTEFOSSE ; et les extraits de Solanum tuberosum commercialisés par la société SEDERMA.
   - les agents stimulant la différenciation des kératinocytes tels que par exemple les minéraux tels que le calcium ; un extrait peptidique de lupin tel que celui commercialisé par la société SILAB sous la dénomination commerciale Structurine^{®}; le beta-sitosteryl sulfate de sodium tel que celui commercialisé par la société SEPORGA sous la dénomination commerciale Phytocohésine^{®} ; et un extrait hydrosoluble de maïs tel que celui commercialisé par la société SOLABIA sous la dénomination commerciale Phytovityl^{®} ; l'extrait de bourgeons de hêtre *Fagus sylvatica* commercialisé par la société GATTEFOSSE sous la dénomination commerciale Gatuline^{®} RC ; un extrait peptidique de *Voandzeia substerranea* tel que celui commercialisé par la société Laboratoires Sérobiologiques sous la dénomination commerciale Filladyn LS 9397^{®} ; et les lignanes tels que le sécoisolaricirésinol.
   - les agents stimulant la prolifération des fibroblastes tels que par exemple les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.
   - les agents stimulant la synthèse du collagène, tels qu'un hydrolysat de protéine de soja commercialisé par SILAB sous la dénomination Nutripeptides, les extraits de *Centella asiatica,* notamment le madécassoside, les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine^{®} ; et les hormones végétales telles que les auxines et les lignanes ;
7) les agents diminuant ou inhibant l'activité des protéases néfastes, tels que les agents agissant :
   - soit sur l'inhibition des métalloprotéinases (métalloprotéinases matricielles ou MMP) telles que plus particulièrement les MMP 1, 2, 3, 9. On peut citer : les rétinoïdes et dérivés, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB^{®}), de trèfle rouge (commercialisé par exemple par la société SEDERMA sous la dénomination « STEROCARE^{®}» ), de lin, de kakkon ou de sauge ; les extraits de cucurma longa ; les extraits de Siegesbeckia (commercialisé par exemple par la société Sederma) ;
   - soit sur l'inhibition de certaines sérine protéases telles que l'élastase leucocytaire ou la cathepsine G. On peut citer : l'extrait peptidique de graines de légumineuse (Pisum sativum) commercialisé par les Laboratoires Sérobiologiques sous la dénomination commerciale Parelastyl^{®}; les héparinoïdes ; un extrait de peptides de riz tel que la Colhibin^{®} de Pentapharm ; et les pseudodipeptides tels que l'acide {2-[acétyl-(3-trifluorométhyl-phényl)-amino]-3-méthyl-butyrylamino} acétique.
8) des agents favorisant la fonction barrière cutanée, tels que un extrait de *Thermus thermophilus* tel que le Vénucéane^{®} de Sederma, un extrait de rhizome d'igname sauvage *(dioscorea villosa)* tel que l'Actigen Y^{®} d'Active Organics, des extraits de plancton comme l'omega plancton^{®} de Secma, des extraits de levure comme le Relipidium^{®} de Coletica, un extrait de chataigne tel que la Recoverine^{®} de Silab, un extrait de bourgeon de cèdre tel que le Gatuline Zen^{®} de Gattefossé, des sphingosines comme la salicyloyl sphingosine vendue sous la dénomination « Phytosphingosine^{®} SLC » par la société Degussa, un mélange de xylitol, de xylityl polyglycoside et de xylitan comme l'Aquaxyl^{®} de Seppic, des extraits de solanacée comme le Lipidessence^{®} de Coletica ; l'hydroxyapatite, en particulier les Hydroxysomes^{®} de la société LSC, et leurs mélanges.
9) des agents tenseurs, tels que par exemple des protéines végétales, en particulier des protéines de soja (ex : Eleseryl^{®} commercialisé par SILAB), des particules colloïdales de charge minérale en particulier des particules colloïdale de silice, des polymères synthétiques, en particulier les latex.
10) des agents apaisants, tels que par exemple l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra) ; l'acide ursolique et ses sels ; les extraits de Centella asiatica, l'huile de Canola, le bisabolol ; les extraits de camomille, l'allantoïne ; un mélange d'extrait de fleur de nénuphar et de palmitoylproline tel que celui vendu sous la dénomination « Seppicalm VG^{®}» par la société Seppic ; l'aloe vera, l'eau de rose, un extrait de menthe, en particulier de feuilles de menthe comme le Calmiskin^{®} de Silab, les bactéries filamenteuse comme Vitreoscilla filiformis tel que décrit dans le brevet EP 761 204, un extrait de pétales de rose comme le Rose Flower Herbasol^{®} extract de la société Cosmetochem, le beurre de karité, et leurs mélanges ;
11) des agents anti-séborrhéiques, tels que notamment
   - les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ; et de préférence le pyrrolidone carboxylate de zinc (ou pidolate de zinc) ou le salicylate de zinc ; le potassium Alum.
   - l'extrait de clou de girofle tel que celui vendu sous la dénomination Clove extract Powder par la société Maruzen ;
   - la glycine greffée sur chaîne undécylénique, telle que celle vendue sous la dénomination Lipacide UG OR par la société Seppic ;
   - le citrate de trialkyle(C₁₂-C₁₃) vendu sous la dénomination COSMACOL® ECI par la société Sasol ; le citrate de trialkyle(C₁₄-C₁₅) vendu sous la dénomination COSMACOL® ECL par la société Sasol.

Le ou les actifs additionnels sont généralement présents dans les compositions cosmétiques en une teneur allant de 0,001 à 10% en poids par rapport au poids total de la composition, de préférence de 0,01 à 5% et encore plus préférentiellement de 0,1 à 2% en poids par rapport au poids total de la composition.

L'invention concerne également une composition cosmétique de soin et/ou de nettoyage de la peau comprenant, dans un milieu physiologiquement acceptable, (i) au moins de l'acide anisique ou l'un de ses dérivés et (ii) de 0.001 à 10% en poids par rapport au poids total de la composition d'au moins un autre agent desquamant distinct de l'acide citrique.

Des exemples d'agents desquamants utilisables dans la composition selon l'invention sont décrits ci-dessus.

En particulier, l'agent desquamant est choisi parmi les α-hydroxyacides, les β-hydroxyacides, l'acide (N-2 hydroxyéthylpiperazine-N-2-éthane) sulfonique (HEPES) ; les dérivés de l'acide 2-oxothiazolidine-4-carboxylique (procystéine) ; l'urée et ses dérivés, l'acide dioïque et ses dérivés.

Un agent desquamant préféré est l'acide n-octanoyl 5-salicylique.

L'acide anisique ou l'un de ses dérivés sera présent dans la composition en une teneur allant de 0.001 à 50% en poids par rapport au poids total de la composition, de préférence de 0.01 à 5% en poids par rapport au poids total de la composition, encore plu préférentiellement de 0.05 à 2% en poids par rapport au poids total de la composition.

Pour des applications de peeling, la composition selon l'invention pourra comprendre des teneurs en acide anisique ou l'un de ses dérivés allant de 1 à 50% en poids par rapport au poids total de la composition, de préférence de 1 à 30% en poids et encore plus préférentiellement de 5 à 20% en poids par rapport au poids total de la composition.

Selon un mode préféré, la composition selon l'invention comprend en outre, en plus de l'acide anisique et de l'autre agent desquamant, au moins un autre actif cosmétique choisi parmi des agents hydratants, des agents antioxydants, des agents dépigmentants, des agents stimulant le métabolisme énergétique des cellules, des agents stimulant la prolifération ou la différenciation épidermique ou stimulant la synthèse de macromolécules épidermiques ou dermiques, des agents diminuant ou inhibant l'activité de protéases néfastes, des agents favorisant la fonction barrière cutanée, des agents tenseurs, des agents apaisants, et des agents antiséborrhéiques.

Des exemples de tels agents additionnels sont décrits précédemment dans la description.
Comme agents additionnels préférés, on peut citer notamment : l'urée et ses dérivés, en particulier le N-(2-hydroxyéthyl)-urée disponible dans le commerce, sous forme de mélange à 50% en poids dans l'eau, auprès de la société NATIONAL STARCH sous la dénomination commerciale Hydrovance^{®}, les céramides ; les dérivés C-glycosides tels que ceux décrits dans EP 1 345 919, en particulier le C-β-D-xylopyranoside-2-hydroxy-propane commercialisé par la société Chimex sous la dénomination Mexoryl SBB^{®}; les tocophérols ; l'adénosine ; des protéines de soja ; des protéines de riz ; un extrait de malt ; un extrait de bourgeon de hêtre ; un extrait de maïs ; l'acide ellagique, l'acide ascorbique et ses dérivés, la biotine, l'hydroxyapatite, l'allantoine, et un extrait de menthe.
En particulier, la composition selon l'invention contiendra au moins 2 agents additionnels distincts, de préférence au moins 3 et encore plus préférentiellement au moins 4.

Ces agents additionnels sont généralement présents dans la composition en une teneur allant de 0.001 à 10% en poids par rapport au poids total de la composition.

Les compositions de soin et/ou de nettoyage selon l'invention contenant l'acide anisique seront avantageuses pour le soin et le traitement de toutes les peaux.
En particulier des compositions seront utilisées pour le soin et/ou le nettoyage des peaux âgées, des peaux à l'aspect terne, des peaux à l'aspect rugueux, des peaux sèches, des peaux grasses, ou pour le soin et le traitement des personnes présentant ce type de peaux.

L'invention concerne également un procédé de traitement cosmétique destiné à favoriser la desquamation et/ou stimuler le renouvellement épidermique, comprenant l'application sur la peau d'une composition comprenant, dans un milieu physiologiquement acceptable, au moins de l'acide anisique ou l'un de ses dérivés.

L'invention porte encore sur un procédé de traitement cosmétique destiné à favoriser l'éclat du teint et/ou diminuer les irrégularités de surface de la peau, comprenant l'application sur la peau d'une composition comprenant, dans un milieu physiologiquement acceptable, au moins de l'acide anisique ou l'un de ses dérivés.

Par irrégularités de surface, on entend notamment les irrégularités visibles et/ou tactiles à la surface de la peau telles que les tâches de vieillesse, les rides et ridules, la visibilité accrue des pores, l'aspect rugueux de la peau, ou les marques d'acné ou de varicelle.

Selon un mode particulier, on applique sur la peau une composition telle que que définie précédemment, comprenant au moins de l'acide anisique et au moins un autre agent desquamant.

Selon un mode particulier, la composition est appliquée sur une peau âgée.
Par 'peaux âgées', on entend des peaux présentant des signes de vieillissement cutané tels que des rides et des ridules, et/ou une perte d'élasticité et/ou de souplesse et/ou de fermeté, notamment au niveau du visage.

Ces peaux caractérisent notamment les personnes de plus de 25 ans, en particulier de plus de 30 ans. On parlera de peaux matures pour les personnes ayant au moins 40 ans, et de peaux très matures pour les personnes ayant au moins 50, voire au moins 60 ans.

Selon un autre mode, la composition est appliquée sur une peau à l'aspect terne et/ou rugueux.

Selon encore un autre mode, la composition est appliquée sur une peau grasse.

Enfin, l'utilisation de l'acide anisique ou l'un de ses dérivés selon la présente invention trouve une autre application dans le domaine des peelings chimiques.

Les peelings sont un moyen bien connu pour améliorer l'aspect et/ou la texture de la peau et/ou du cuir chevelu, notamment améliorer l'éclat et l'homogénéité du teint et/ou diminuer les irrégularités visibles et/ou tactiles de la peau, et en particulier pour améliorer l'aspect de surface de la peau, pour atténuer les lentigo actiniques, les marques d'acné ou de varicelle, ainsi que pour prévenir, atténuer ou lutter contres les signes du vieillissement cutané, et notamment pour lisser les irrégularités de la texture de la peau, telles les rides et les ridules.
Ils ont pour effet d'enlever une partie superficielle de la peau à traiter (épiderme et éventuellement couche superficielle du derme), par des méthodes chimiques.

Rentre donc également dans l'invention un procédé de traitement cosmétique des irrégularités visibles et/ou tactiles de la peau humaine, comprenant les étapes consistant à :
a) appliquer topiquement sur la peau une composition contenant de l'acide anisique ou l'un de ses dérivés ;
b) laisser la composition au contact de la peau pendant une durée comprise entre 5 mn et 6 heures, de préférence entre 5 mn et 30 mn, et
c) éliminer la composition par rinçage.

La teneur en acide anisique ou l'un de ses dérivés dans la composition de peeling sera généralement d'au moins 1% et de préférence ira de 1% à 50%, en particulier de 1 à 30% et préférentiellement de 5 à 20% en poids par rapport au poids total de la composition.

Ce procédé est plus particulièrement destiné à prévenir, atténuer, et/ou lutter contre les signes du vieillissement cutané, et notamment atténuer les rides et les ridules, et à améliorer l'aspect et/ou la texture de la peau, en particulier à améliorer l'homogénéité du teint et/ou lisser la peau, et notamment atténuer des lentigo actiniques ou autres désordres esthétiques pigmentaires (ex : tâches brûnatres), ou des hyperpigmentations localisées, ou des marques d'acné ou de varicelle, et/ou désobstruer les pores de la peau.

L'invention va maintenant être illustrée par les exemples non limitatifs suivants.

### EXEMPLES

### Exemple 1 : Mise en évidence d'un effet desquamant de l'acide anisique

Les propriétés desquamantes de l'acide anisique ont été mises en évidence sur un modèle de peau maintenue en survie, par analyse de la cohésion du *stratum corneum* sur coupes histologiques colorées à l'hemalun-eosine.
Des fragments de peau humaine normale sont obtenus en chirurgie plastique de 6 donneurs différents. Ils sont déposés dans des inserts eux-mêmes positionnés sur des puits de culture, contenant du milieu de culture spécifiquement adapté au maintien en survie (antibiotiques, SVF) desdits fragments de peau.
L'acide anisique est appliqué topiquement selon le protocole suivant et en comparaison avec une peau non traitée (peau témoin) :
- peau témoin
- peau + acide anisique à 0,1%
- peau + acide anisique à 1 %

On a évalué la cohésion de la couche cornée à partir de coupes histologiques colorées, selon des scores semi-quantitatifs suivants (évaluation sur 10 à 15 champs au grossissement 40) :

| | |
|---|---|
| score 0 | absence de modification de la cohésion du *stratum corneum* |
| score 1 | diminution légère de la cohésion du *stratum corneum* |
| score 2 | diminution modérée de la cohésion *du stratum corneum* |
| score 3 | diminution importante de la cohésion *du stratum corneum* |
| score 4 | diminution très importante de la cohésion du *stratum corneum* avec exfoliation. |

Une moyenne est réalisée pour chaque paramètre à partir des résultats obtenus sur les 6 modèles de peaux. L'analyse statistique est effectuée par le test de Student dit de l'écart réduit ou test des échantillons appariés, avec un risque de 5%.

Les résultats sont présentés dans le tableau suivant :

**Tableau : Evaluation histologique de la cohésion de la couche cornée après coloration par l'hemalun-éosine (scores semi-quantitatifs).**

| **Traitement** | | **Scores** | **% d'augmentation de la desquamation par rapport à la peau témoin** |
|---|---|---|---|
| Témoin | | 0,73 ± 0,2 | 0,0% |
| Acide Anisique (1) | 0,1% | 1,43 ± 0,7* | 95,9% |
| | 1% | 1,57 ± 0,4* | 115% |

| | | | |
|---|---|---|---|
| (1) : acide para-anisique ou acide 4-methoxybenzoïque ou acide para methoxybenzoïque, commercialisé par la société MERCK. *: différence statistiquement significative par rapport à la peau témoin (test de Student apparié, p < 0,05). | | | |

Dans les conditions expérimentales de cette étude, le traitement des peaux par l'acide anisique à 0,1 % et 1 % montre une diminution significative de la cohésion du *stratum corneum* par rapport à la peau témoin, avec des scores respectifs de 1,43 et 1,57 contre 0,73 pour la peau témoin (plus le score est élevé, plus la cohésion diminue), ce qui se traduit par une augmentation de la desquamation de +95,9 % et + 115 % respectivement.

Ces résultats mettent en évidence une activité desquamante significative après application topique d'acide anisique par rapport aux peaux témoins, se traduisant par une diminution histologique de la cohésion du *stratum corneum,* sans toutefois montrer d'effet dose.

### Exemple 2 : Formulations

### Crème de jour

| | |
|---|---|
| Acide p-anisique | 0.5% |
| acide n-octanoyl 5-salicylique | 0.1 % |
| protéine de soja | 5% |
| protéine de riz | 1% |
| Corps gras solides | 3% |
| Alcools gras | 3% |
| Silice | 4% |
| Cylcohexasiloxane | 4% |
| Glycérine | 5% |
| Emulsionnants | 7% |
| Conservateurs | 0.5% |
| Eau | qsp 100% |

La composition est appliquée quotidiennement sur le visage pour un effet de lissage du microrelief cutané.

| Crème de protection solaire | |
|---|---|
| Acide p-anisique | 0,25% |
| Glycerine | 7% |
| Cyclopentasiloxane | 10% |
| Corps gras solides | 3% |
| Huile vegetale | 3% |
| Gelifiant | 2% |
| Alcools gras | 1% |
| Acides gras | 3% |
| Filtres UV | 10% |
| PEG-100 stearate | 2% |
| Conservateurs | 0.5% |
| Alcool | 5% |
| Eau | qsp 100% |

Cette crème est appliquée sur le visage le matin pour favoriser l'éclat du teint et protéger la peau contre les effets des UV.

| Crème démaquillante | |
|---|---|
| Acide p-anisique | 0.2% |
| Silice | 1% |
| Ethyhexyl palmitate | 66% |
| Alcool | 2% |
| Glycérine | 10% |
| Beheneth-10 | 5% |
| Conservateurs | 0,55% |
| Eau | qsp 100% |

Cette crème démaquillante appliquée sur la peau du visage permet de nettoyer en douceur la peau, diminuer la visibilité des pores et améliorer le grain de la peau.

| Savon moussant | |
|---|---|
| Acide p-anisique | 0.1% |
| Potassium hydroxyde | 6 % |
| Sorbitol | 7% |
| Corps gras | 4% |
| Glycérine | 14% |
| Tensioactifs | 34% |
| Eau | qsp 100% |

Ce savon moussant utilisé quotidiennement sur la peau permet de désobstruer les pores et redonne à la peau un aspect de teint homogène.

| Composition de peeling anti-rides | |
|---|---|
| Acide p-anisique | 5% |
| Acide Glycolique | 10% |
| HEPES | 5% |
| Gélifiant | 0.5% |
| Glycérine | 10 % |
| ALCOOL | 20% |
| Eau purifiée ou eau thermale | qsp 100 % |

Cette composition peut être appliquée sous forme de peeling pour atténuer les rides et ridules faciales, et/ou améliorer l'éclat du teint.

## Revendications

1. Utilisation cosmétique d'une composition contenant, dans un milieu physiologiquement acceptable, de l'acide anisique, l'un de ses sels ou l'un de ses esters d'alkyle en C₁-C₄, pour favoriser la desquamation de la peau et/ou stimuler le renouvellement épidermique.

2. Utilisation cosmétique, dans une composition contenant un milieu physiologiquement acceptable, de l'acide anisique, l'un de ses sels ou l'un de ses esters d'alkyle en C₁-C₄, comme agent destiné à favoriser la desquamation de la peau et/ou stimuler le renouvellement épidermique.

3. Utilisation cosmétique d'une composition contenant, dans un milieu physiologiquement acceptable, de l'acide anisique, l'un de ses sels ou l'un de ses esters d'alkyle en C₁-C₄, pour améliorer au moins une des conditions choisie parmi :
- améliorer l'éclat et/ou l'homogénéité du teint et/ou diminuer l'aspect de teint brouillé et/ou terne ;
- améliorer l'aspect de surface, en particulier diminuer l'aspect rugueux ou craquelé de la peau et/ou améliorer le grain et/ou la douceur de la peau ;
- lisser le microrelief de la peau, en particulier lisser les ridules et les rides et/ou atténuer les marques d'acné ou de varicelle ;
- atténuer les tâches de vieillesse et/ou les désordres esthétiques de pigmentation ;
- diminuer la sécheresse de la peau, en particulier la sécheresse de la peau âgée ;
- améliorer l'apparence et/ou diminuer la visibilité des pores et/ou améliorer la désobstruction des pores ;
- favoriser l'action de nettoyage et l'élimination de cellules mortes à la surface de la peau ; et/ou
- lutter contre les imperfections des peaux grasses, en particulier l'aspect brillant ou luisant de la peau.

4. Utilisation cosmétique, dans une composition contenant un milieu physiologiquement acceptable, de l'acide anisique, l'un de ses sels ou l'un de ses esters d'alkyle en C₁-C₄ en tant que tel, comme agent destiné à améliorer au moins une des conditions choisie parmi :
- améliorer l'éclat et/ou l'homogénéité du teint et/ou diminuer l'aspect de teint brouillé et/ou terne ;
- améliorer l'aspect de surface, en particulier diminuer l'aspect rugueux ou craquelé de la peau et/ou améliorer le grain et/ou la douceur de la peau ;
- lisser le microrelief de la peau, en particulier lisser les ridules et les rides et/ou atténuer les marques d'acné ou de varicelle ;
- atténuer les tâches de vieillesse et/ou les désordres esthétiques de pigmentation ;
- diminuer la sécheresse de la peau, en particulier la sécheresse de la peau âgée ;
- améliorer l'apparence et/ou diminuer la visibilité des pores et/ou améliorer la désobstruction des pores ;
- favoriser l'action de nettoyage et l'élimination de cellules mortes à la surface de la peau ; et/ou
- lutter contre les imperfections des peaux grasses, en particulier l'aspect brillant ou luisant de la peau.

5. Utilisation selon l'une quelconque des revendications précédentes, **caractérisée en ce que** l'acide anisique, l'un de ses sels ou l'un de ses esters d'alkyle en C₁-C₄ est présent dans la composition en une quantité allant de 0.001 à 50% en poids par rapport au poids total de la composition, de préférence de 0.01 à 5% en poids par rapport au poids total de la composition, encore plus préférentiellement de 0.05 à 2% en poids par rapport au poids total de la composition.

6. Utilisation selon l'une quelconque des revendications précédentes, **caractérisée en ce que** la composition est destinée au soin et/ou au nettoyage des peaux âgées.

7. Utilisation selon l'une quelconque des revendications précédentes, **caractérisée en ce que** la composition est destinée au soin et/ou au nettoyage des peaux à l'aspect terne et/ou rugueux.

8. Utilisation selon l'une quelconque des revendications précédentes, **caractérisée en ce que** la composition est destinée au soin et/ou au nettoyage des peaux grasses.

9. Utilisation selon l'une quelconque des revendications précédentes, **caractérisée en ce que** la composition comprend en outre au moins un autre actif cosmétique choisi parmi d'autres agents desquamants, des agents hydratants, des agents antioxydants, des agents dépigmentants, des agents stimulant le métabolisme énergétique des cellules, des agents stimulant la prolifération ou la différenciation épidermique ou stimulant la synthèse de macromolécules épidermiques ou dermiques, des agents diminuant ou inhibant l'activité de protéases néfastes, des agents favorisant la fonction barrière cutanée, des agents tenseurs, des agents apaisants, des agents antiséborrhéiques, et leurs mélanges.

10. Utilisation selon la revendication 9, **caractérisée en ce que** le (ou les) autre(s) actif(s) est présent dans la composition en une teneur allant de 0.001 à 10% en poids par rapport au poids total de la composition.

11. Utilisation selon l'une quelconque des revendications précédentes, **caractérisé en ce que** l'acide anisique est l'acide para-anisique ou acide 4-methoxybenzoïque ou acide para-methoxybenzoïque.

12. Composition cosmétique de soin et/ou de nettoyage de la peau comprenant, dans un milieu physiologiquement acceptable, (i) au moins de l'acide anisique, l'un de ses sels ou l'un de ses esters d'alkyle en C₁-C₄ et (ii) de 0.001 à 10% en poids par rapport au poids total de la composition d'au moins un autre agent desquamant distinct de l'acide citrique.

13. Composition selon la revendication 12, **caractérisée en ce que** l'agent desquamant est choisi parmi les α-hydroxyacides, les β-hydroxyacides, l'acide (N-2 hydroxyéthylpiperazine-N-2-éthane) sulfonique (HEPES) ; les dérivés de l'acide 2-oxothiazolidine-4-carboxylique (procystéine) ; l'urée et ses dérivés, l'acide dioïque et ses dérivés.

14. Composition selon l'une des revendications 12 ou 13, **caractérisée en ce que** l'agent desquamant est l'acide n-octanoyl 5-salicylique.

15. Composition selon l'une des revendications 12 à 14, **caractérisée en ce que** l'acide anisique, l'un de ses sels ou l'un de ses esters d'alkyle en C₁-C₄ est présent dans la composition en une teneur allant de 0.001 à 10% en poids par rapport au poids total de la composition.

16. Composition selon l'une quelconque des revendications 12 à 15, **caractérisée en ce qu'**elle comprend en outre au moins un autre actif cosmétique choisi parmi des agents hydratants, des agents antioxydants, des agents dépigmentants, des agents stimulant le métabolisme énergétique des cellules, des agents stimulant la prolifération ou la différenciation épidermique ou stimulant la synthèse de macromolécules épidermiques ou dermiques, des agents diminuant ou inhibant l'activité de protéases néfastes, des agents favorisant la fonction barrière cutanée, des agents tenseurs, des agents apaisants, et des agents antiséborrhéiques.

17. Procédé de traitement cosmétique destiné à favoriser la desquamation et/ou stimuler le renouvellement épidermique, comprenant l'application sur la peau d'une composition comprenant, dans un milieu physiologiquement acceptable, au moins de l'acide anisique, l'un de ses sels ou l'un de ses esters d'alkyle en C₁-C₄.

18. Procédé de traitement cosmétique pour favoriser l'éclat du teint et/ou diminuer les irrégularités de surface de la peau en particulier les tâches de vieillesse, les rides et ridules, la visibilité accrue des pores, ou l'aspect rugueux de la peau, comprenant l'application sur la peau d'une composition comprenant, dans un milieu physiologiquement acceptable, au moins de l'acide anisique, l'un de ses sels ou l'un de ses esters d'alkyle en C₁-C₄.

19. Procédé selon l'une quelconque des revendications 17 ou 18, **caractérisé en ce que** l'on applique sur la peau une composition telle que définie dans l'une quelconque des revendications 12 à 16.

20. Procédé selon l'une quelconque des revendications 17 à 19, **caractérisé en ce que** la composition est appliquée sur une peau âgée.

21. Procédé selon l'une quelconque des revendications 17 à 19, **caractérisé en ce que** la composition est appliquée sur une peau à l'aspect terne et/ou rugueux.

22. Procédé de traitement cosmétique des irrégularités visibles et/ou tactiles de la peau humaine, comprenant les étapes consistant à :
a) appliquer topiquement sur la peau une composition contenant de l'acide anisique, l'un de ses sels ou l'un de ses esters d'alkyle en C₁-C₄;
b) laisser la composition au contact de la peau pendant une durée comprise entre 5 mn et 6 heures, de préférence entre 5 mn et 30 mn, et
c) éliminer la composition par rinçage.
